# EUROPÄISCHE PATENTSCHRIFT

(11) **EP 2 644 167 B1**
(45) Veröffentlichungstag und Bekanntmachung des Hinweises auf die Patenterteilung: **07.01.2015**
(21) Anmeldenummer: 13160582.6
(22) Anmeldetag: 22.03.2013
(51) Int. Cl.: A61F 2/66, A61F 2/50

(54) **Fußprothese**
Foot prosthesis
Prothèse du pied

(30) Priorität: 27.03.2012 DE 102012006023
(43) Veröffentlichungstag der Anmeldung: 02.10.2013
(73) Patentinhaber: medi GmbH & Co. KG, 95448 Bayreuth (DE)
(72) Erfinder: Kranner, Werner, 83043 Bad Aibling (DE); Nissels, Volker, 95445 Bayreuth (DE)
(74) Vertreter: Blaumeier, Jörg

(56) Entgegenhaltungen:
- EP-A1- 2 420 212
- WO-A2-02/30340
- US-A1- 2003 009 238
- US-A1- 2006 185 703
- US-A1- 2011 029 097
- US-B1- 6 852 132

## Beschreibung

Die Erfindung betrifft eine Fußprothese, umfassend ein Oberteil und ein beim Laufen bodenseitig aufsetzendes Unterteil, die übereinander und voneinander beabstandet verlaufen, an der Fußspitze und der Ferse miteinander verbunden sind, und, eine Feder bildend, beim Laufen relativ zueinander bewegen, wobei das Oberteil und das Unterteil mittels eines einstückigen, geschlossenen Bauteils aus einem Kohlefaserverbundwerkstoff gebildet sind.

Eine Fußprothese der beschriebenen Art ermöglicht dem Träger ein komfortables und aufgrund der Federeigenschaften der Prothese gedämpftes Auftreten sowie ein dem realen Fuß angenähertes Laufen. Moderne Fußprothesen bestehen beispielsweise aus einem Carbonlaminat, also einem Kohlefaserverbundwerkstoff. Sie weisen ein Oberteil auf, über das die Fußprothese mit einem Verbindungsschaft oder ähnlichem, mit dem die gesamte Prothese am Beinstumpf befestigt wird, über ein geeignetes Verbindungsmittel wie beispielsweise eine Pyramidenaufnahme gekoppelt wird. Das Oberteil erstreckt sich letztlich vom Fersenbereich bis zur Fußspitze. Im Bereich der Fußspitze ist das Oberteil mit einem Unterteil verbunden, das beim Laufen bodenseitig aufsetzt. Oberteil und Unterteil sind voneinander beabstandet, liegen jedoch näherungsweise deckungsgleich übereinander. Üblicherweise sind Ober- und Unterteil im Fersenbereich über ein elastisches Dämpfungselement, beispielsweise aus einem gummiartigen Material, das als Auftrittsdämpfer wirkt und die beim Aufsetzen wirkende Kraft dämpft, verbunden. D. h., dass Ober- und Unterteil fersenseitig übereinander enden und über das Dämpfungselement gekoppelt sind. Im Bereich zwischen diesem Dämpfungselement und der Fußspitze bilden das Ober- und Unterteil eine Feder, die beim Laufen gebogen und beansprucht wird. Im Bereich der Fußspitze können das Ober- und Unterteil über ein separates Verbindungselement miteinander verbunden sein, beispielsweise unter Integration einer dämpfenden Zwischenlage miteinander verschraubt sein, bekannt ist es auch, das Ober- und Unterteil über eine Umlenkung an der Fußspitze direkt zu verbinden.

Das Ober- und Unterteil bilden zusammen letztlich eine Feder, die bei Belastung einfedert und so das komfortable Laufen ermöglicht. Das Fersenelastomer wirkt als Auftrittsdämpfer und Drehpunkt zwischen Rückfuß- und Vorderfußhebel, also zwischen Fersenbereich und Fußspitzenbereich. Für eine feste Verbindung ist das Fersenelastomer bei bekannten Prothesen relativ großflächig mit dem Ober- und Unterteil verbunden, üblicherweise verklebt. Hierüber kann zwar eine feste Integration des dampfenden Fersenelastomers erreicht werden, das ja beachtlich beansprucht wird und für lange Zeit seine Funktion ausführen soll. Jedoch kommt es durch die Integration dieses Fersendämpfungselements, zumeist ein Elastomerblock aus einem eine definierte Shore-A-Härte aufweisenden Elastomer, zu einer Verkürzung der effektiven Federlängen im Bereich des Ober- und Unterteils respektive der Federwege und dadurch zu einer Verringerung der Verformungsmöglichkeiten. Verstärkt wird dieser Effekt, wenn Ober- und Unterteil auch im Bereich der Fußspitze beispielsweise über eine Verschraubung oder Verklebung miteinander verbunden sind, d. h., dass dann noch kürzere Federlängen respektive Federwege gegeben sind.

Eine Fußprothese der eingangs genannten Art ist aus US 6852132 B1 bekannt. Eine Ausführungsform der dort beschriebenen Prothesen besteht aus einer unteren, quasi ringförmigen Feder aus einem Kohlefaserverbundwerkstoff, auf die ein zweites elastisches, quasi U-förmiges Trägerteil aufgesetzt ist, das eine Aufnahme für den Gliedmaßenstumpf aufweist.

Der Erfindung liegt damit das Problem zugrunde, eine Fußprothese anzugeben, die demgegenüber verbessert ist und eine verbesserte Beweglichkeit respektive Federeigenschaft von Oberteil- und Unterteil ermöglicht.

Zur Lösung dieses Problems ist bei einer Fußprothese der eingangs genannten Art erfindungsgemäß vorgesehen, dass das Oberteil und das Unterteil über jeweils eine im Bereich zwischen 135° - 180° verlaufende Umlenkung an der Fußspitze und der Ferse ineinander übergehen.

Bei der erfindungsgemäßen Fußprothese kommt zur Bildung des eigentlichen Prothesenkörpers, nämlich der Feder bestehend aus Ober- und Unterteil nur ein einstückiges, geschlossenes Bauteil zum Einsatz, das aus einem Kohlefaserverbundwerkstoff, also einem Carbonlaminat besteht. Dieses Bauteil benötigt folglich keine speziellen Verbindungselemente zwischen Ober- und Unterteil, da diese einteilig am einstückigen, geschlossenen Carbonbauteil ausgeformt sind. Sie sind über geeignete Umlenkungen miteinander verbunden, sodass sich einerseits die Federeigenschaft ergibt, andererseits auch die entsprechende Relativbeweglichkeit beider zueinander, die für eine reaktionskraftbedingte Verformung beim Laufen erforderlich ist. Nachdem keine Verbindungselemente zur Kopplung von Ober- und Unterteil erforderlich sind, kann folglich die maximale Federlänge respektive größtmögliche Federwege effektiv genutzt werden, d. h., dass sich der Sohlenbereich, also der Bereich des Unterteils, über die gesamte Fußlänge homogen verformen und auch tordieren kann, wodurch ein homogenes Abrollen gewährleistet werden kann, da sich im Verlauf des Schrittzyklus die Biegesteifigkeit der sich momentan im Bodenkontakt befindlichen Bereichs der Feder, also des Bauteils nicht sprunghaft ändert. Die Möglichkeit, die maximalen Federlängen respektiven Federwege nutzen zu können, führt dazu, dass ein hinreichende Torsionsweichheit erreicht wird, was wiederum zu einer ausgewogenen Pro- und Supinationsmöglichkeit des Prothesenfußes führt und eine gute Anpassung an verschiedenen Untergründe und Bodenunebenheiten ermöglicht, anders als bei den über die Verbindungselemente quasi fest verspannten Ober- und Unterteilkonfigurationen.

Die erfindungsgemäße Fußprothese besteht also nur aus einer Feder, nämlich dem einstückigen, geschlossenen Bauteil und bietet dem Nutzer mit hoher Mobilität eine gute Funktionalität und Energierückgabe. Denn der besondere Vorteil der erfindungsgemäßen Fußprothese mit der einteiligen Feder besteht in einer ununterbrochenen Federwirkung, d. h., dass der Energiefluss nicht durch starre Verbindungspunkte wie Verschraubungen, Verklebungen, Laminierungen, wie sie im Stand der Technik durch die Integration zumindest des Fersendämpfungselements, gegebenenfalls auch der spitzenseitigen Verschraubung, gegeben sind, unterbrochen wird.

Das Oberteil und das Unterteil gehen wie beschrieben einstückig im Feder-Bauteil ineinander über. Dies wird erfindungsgemäß über jeweils eine im Bereich zwischen 135° - 180° verlaufende Umlenkung an der Fußspitze und der Ferse realisiert.

Eine besonders zweckmäßige Weiterbildung der Erfindung sieht vor, dass das Bauteil im fersennahen Bereich des Oberteils eine weitere Umlenkung aufweist, derart, dass ein oberhalb des Unterteil verlaufender erster Oberteilabschnitt über die Umlenkung in einen oberhalb des ersten Abschnitts verlaufenden zweiten Oberteilabschnitt übergeht. Über diese zweite Umlenkung, die oberteilseitig vorgesehen ist, wird quasi ein weiterer Federabschnitt realisiert. Ein erster Federabschnitt ist bereits über die Umlenkung vom Oberteil zum Unterteil realisiert, also über die unmittelbare Verbindung beider Teile. Das Oberteil ist nun erfindungsgemäß nochmals in sich unter Bildung einer weiteren Umlenkung gebogen, sodass zwei Oberteilabschnitte übereinander verlaufen und sich hierdurch ein weiterer Federabschnitt bildet. Es ergibt sich an diesem Oberteilbereich letztlich eine S-Form. Beim Laufen kommt es auch im Bereich dieser weiteren Umlenkung zu einer Einfederung, mithin also einer Energieeinspeicherung respektive einem Ausfedern und einer Energierückgabe. Dabei kann im Bereich der Umlenkung an einem der beiden oder beiden Oberteilabschnitten ein Elastomerelement eingebracht respektive angeordnet sein. Dieses Elastomerelement dient der Dämpfung der Einfederbewegung, d. h., dass bei hinreichend weiter Einfederung sich der eine Oberteilabschnitt an das Elastomerelement am anderen Oberteilabschnitt anlegt und die weitere Einfederbewegung zusätzlich über das Elastomerelement gedämpft wird.

Zur weiteren gezielten Dämpfung der Einfederbewegung sieht eine Weiterbildung der Erfindung vor, im Bereich zwischen dem Oberteil und dem Unterteil ein die Umlenkung, die am fersennahen Oberteilbereich mittels der beiden Oberteilabschnitte realisiert ist, umgreifendes, zumindest beim Laufen das Oberteil mit dem Unterteil koppelndes Elastomerelement vorgesehen ist, wobei dieses Elastomerelement vorzugsweise lösbar einsetzbar ist. Wie beschrieben ist das erfindungsgemäße, die Fußprothese bildende Bauteil ein geschlossenes Bauteil. Wird der fersennahe Oberteilbereich wie beschrieben über die Umlenkung als eigener Federabschnitt ausgebildet, mithin also quasi S-förmig ausgeformt, so kann nun gemäß dieser Erfindungsausgestaltung im "Bauteilinneren", die Umlenkung umgreifend, ein weiteres Elastomerelement integriert werden. Beim Laufen federt das Oberteil relativ zum Unterteil ein, wenn die Prothese belastet wird. Sie nähern sich also einander an. Diese Annäherungsbewegung kann nun über das weitere Elastomerelement ebenfalls bedämpft werden. Dabei kann je nach Größe des Elastomerelements der Zeitpunkt bestimmt werden, zu dem das Oberteil und das Unterteil über das Elastomerelement gekoppelt werden. Ist das Elastomerelement so groß gewählt, dass bereits bei unbelasteter Prothese das Oberteil und das Unterteil am Elastomerelement anliegen, so ist bei bereits geringer Einfederung eine Dämpfung hierüber gegeben. Ist ein oder sind beide Teile bei unbelasteter Fuß-prothese vom Elastomerelement beabstandet, so tritt die Dämpfungswirkung zwangsläufig erst zu einem späteren Zeitpunkt ein, wenn eben beide hinreichend eingefedert sind.

In Weiterbildung der Erfindung kann das Oberteil im fersennahen Bereich, insbesondere im Bereich des ersten und zweiten Oberteilabschnitts, eine Elastomereinlage im Kohlefaserverbundwerkstoff, die seine Biegeeigenschaften lokal variiert, aufweisen. Der Kohlefaserverbundwerkstoff ist ein Mehrlagenlaminat. Dies ermöglicht es ohne Weiteres, in dieses Laminat eine Elastomerzwischenlage zu integrieren. Diese wird gemäß dieser Erfindungsausgestaltung im fersennahen Bereich des Oberteils realisiert, sie befindet sich bevorzugt im Bereich des ersten und/oder zweiten Oberteilabschnitts, mithin also im Bereich der dortigen Umlenkung. Bevorzugt kann sich diese Elastomereinlage vom ersten in den zweiten Oberteilabschnitt erstrecken, mithin also durch die Umlenkung laufen. Je nachdem, wie dick die Einlage gewählt wird respektive welche Shore-A-Härte sie aufweist, umso weicher oder härter kann die Federeigenschaft respektive Biegesteifigkeit dieses Oberteilbereichs eingestellt werden.

In Weiterbildung der Erfindung kann vorgesehen sein, dass das Bauteil im fersennahen Bereich, insbesondere mit dem zweiten Oberteilabschnitt gebogen in Richtung der Fußspitze verläuft und mit dem von der Fußspitze kommenden Oberteilabschnitt, der im fersennahen Bereich ebenfalls in Richtung der Fußspitze umgebogen verläuft, unter Bildung eines Befestigungsabschnitts für ein Verbindungsmittel, insbesondere eine Pyramidenaufnahme zusammenläuft. Der Befestigungsabschnitt erstreckt sich quasi, bezogen auf den Verlauf des Unterteils, schräg nach oben und ist in Richtung Fußspitze geführt. Um ihn so zu formen, ist es erforderlich, den Oberteilabschnitt, der von der Fußspitze kommend zum Befestigtensabschnitt läuft, sowie den Oberteilabschnitt, der fersennahe ist (bei S-förmiger Ausgestaltung also den zweiten Oberteilabschnitt im fersennahen Bereich) ebenfalls entsprechend gebogen zu führen, sodass beide zusammenlaufen und den Befestigungsabschnitt bilden. Dieser ist hinreichend stark ausgelegt, um eine sichere Fixierungsmöglichkeit für ein Verbindungsmittel wie beispielsweise eine Pyramidenaufnahme, die zur Anbindung des Prothesenschaftes dient, zu bilden. Denkbar ist alternativ hierzu aber auch das Bauteil mit seinem Befestigungsabschnitt vertikal nach oben laufend auszuführen, so dass sich dieser Abschnitt vom oberen Ende her kommend in einen zur Fußspitze laufenden Oberteilabschnitt und einen zur Ferse, dort z. B. in eine Federgeometrie laufenden Oberteilabschnitt verzweigt.

Im Bereich der Verbindung beider Oberteilabschnitte kann dabei ein beide verbindendes Elastomerelement eingesetzt sein.

Schließlich sieht eine zweckmäßige Weiterbildung der Erfindung vor, dass auch im Bereich zwischen dem Oberteil und dem Unterteil, also im "Bauteilinneren" ein Befestigungselement für ein lösbar einsetzbares und beim Laufen das Oberteil mit dem Unterteil koppelndes Elastomerelement vorgesehen ist. Dieses Elastomerelement, das bevorzugt länglich ausgeführt ist, ist also ein Füllstück, das zwischen dem Ober- und Unterteil lösbar eingesetzt wird, mithin also bei Bedarf integriert werden kann, aber auch fest dort integriert sein kann. Dieses Elastomerelement tritt während der Laufbewegung in Kontakt mit einer mit beiden einander zugewandten Flächen des Ober- und Unterteils, je nach Auslegung hinsichtlich Länge und Dicke des Elastomerelements. Hierbei kommt es folglich zu einer Veränderung des Anlagevorgangs oder Kopplungsvorgangs des Oberteils mit dem Unterteil, die erfindungsgemäß über dieses Elastomerelement gekoppelt werden, wenn die Fußprothese belastet wird, mithin als das Oberteil hinreichend weit in Richtung des Unterteils einfedert. Der sich beim Laufen ergebenden Bewegung des Oberteils relativ zum Unterteil wird über das Elastomerelement, an dem beide temporär anliegen, ein Widerstand entgegengesetzt, sodass es zu einer Versteifung der aus Oberteil und Unterteil im Bereich zwischen Ferse und Fußspitze gebildeten Feder kommt. D. h., dass eine die Steifigkeit erhöhende Verbindung nicht erst dann während der Laufbewegung einsetzt, wenn das Oberteil das Unterteil unmittelbar kontaktiert, sondern bereits wesentlich früher, nämlich dann, wenn ein Flächenkontakt über das eingesetzte Elastomer-Füllstück gegeben ist.

Dieser Kopplungszeitpunkt kann durch entsprechende längen- oder dickenmäßige Auslegung dieses bevorzugt flächigen, zungenartigen Elastomerelements, das gegebenenfalls auch strukturiert sein kann (z. B. mit pyramidenartig zulaufenden Erhebungen an einer Seite oder mit einem Wellenprofil), der Kopplungszeitpunkt eingestellt werden, wie natürlich auch durch eine entsprechende Materialwahl die Biegesteifigkeit variiert werden kann. D. h., dass letztlich die Aufhärtung der Prothese insoweit veränderbar ist, was insbesondere für den Anwender vom Vorteil ist, wenn dieses Elastomerelement lösbar eingesetzt werden kann. Denn dann kann der Anwender die Entscheidung treffen, wie er die Härte der Prothese einstellen möchte. Hierüber kann also individuell eingestellt werden, ab welcher Phase des Schrittzyklus die Vorderfußhärte überproportional anzusteigen beginnt.

Um das lösbare Einsetzen möglichst einfach zu gestalten, kann das Befestigungselement einen Rastvorsprung aufweisen, der vorzugsweise zum Unterteil gerichtet ist, und der in eine am einzusetzenden Elastomerelement vorgesehene Rastaufnahme eingreift. D. h., dass der Anwender dieses Elastomerelement auf relativ einfache Weise einrasten oder einklipsen kann. Denkbar ist aber auch die Möglichkeit, über eine entsprechende hintergreifende Formgebung dieses Elastomerelements von der Seite her in eine entsprechende Aufnahme einzuschieben etc.

Das Befestigungselement befindet sich im fersennahen Bereich, bevorzugt am Unterteil. Es befindet sich also, sofern vorgesehen, im Bereich der weiteren Umlenkung am Oberteil, über die der erste Oberteilabschnitt in den zweiten Oberteilabschnitt umgelenkt wird. Ist nun im Bereich dieser Umlenkung, diese umgreifend, ein Elastomerelement vorgesehen, so kann der Befestigungsabschnitt derart ausgeformt sein, dass das umlenkungsseitige Elastomerelement formschlüssig in den Befestigungsabschnitt eingreift, mithin dort also ebenfalls gegengelagert ist, worüber letztlich die Kopplung zum Unterteil gegeben sein kann.

Die Elastomerelemente, sofern sie nicht lösbar sind, können durch geeignete Verklebung am Bauteil angeordnet sein. Da sie lediglich an einem Bauteil fixiert sind, kommt es zwangsläufig nicht zu einer von Haus aus gegebenen Federwegrespektive Federhebelverkürzung, sondern erst, wenn - wie beabsichtigt - das jeweils andere Teil an dem Elastomerelement angreift.

Zur Bildung der Elastomerelemente wird nach Bedarf ein geeignetes Kunststoffmaterial verwendet, das die gewünschte Shore-A-Härte aufweist.

Weitere Vorteile, Merkmale und Einzelheiten der Erfindung ergeben sich aus dem im Folgenden beschriebenen Ausführungsbespiel sowie anhand der Zeichnungen. Dabei zeigen:
Fig. 1 eine Seitenansicht einer erfindungsgemäßen Fußprothese einer ersten Ausführungsform,
Fig. 2 eine Seitenansicht einer erfindungsgemäßen Fußprothese einer zweiten Ausführungsform ohne integrierte, lösbare Elastomerelemente, und
Fig. 3 - 7 die Fußprothese aus Fig. 2 mit unterschiedlichen integrierten Elastomerelementen.

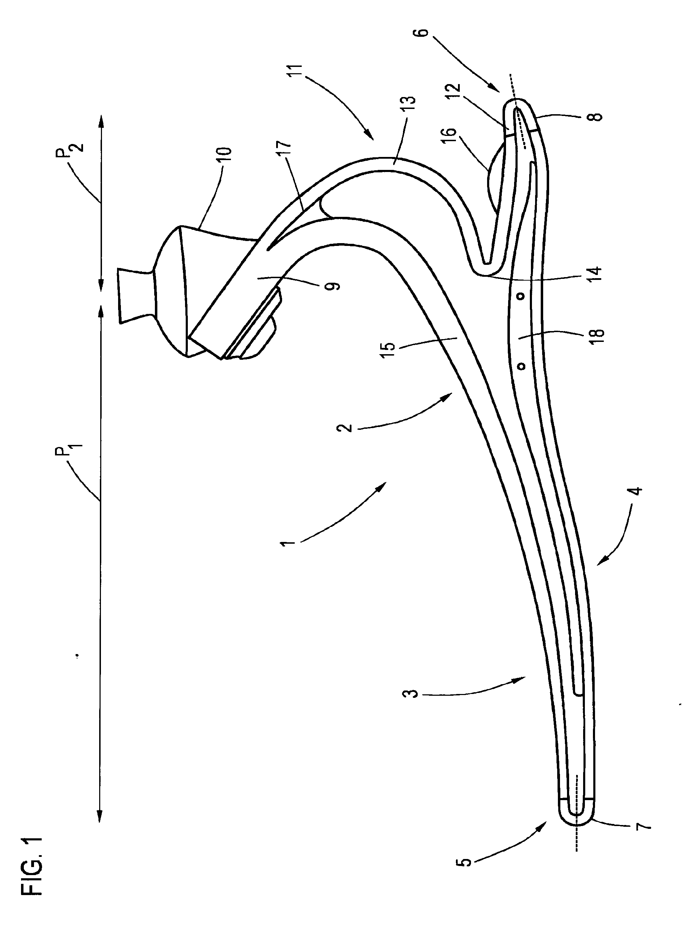

Fig. 1 zeigt eine erfindungsgemäße Fußprothese 1, umfassend ein einstückiges, eine Feder bildendes Bauteil 2 aus einem Kohlefaserverbundwerkstoff, das das zentrale Prothesenbauteil bildet. Am respektive mittels des Bauteils 2 sind zwei grundsätzlich voneinander zu unterscheidende Teile realisiert, nämlich zum einen ein Oberteil 3 sowie ein Unterteil 4, wobei über die beiden gestrichelten Linien angedeutet ist, bis wohin das Oberteil 3 und bis wohin das Unterteil 4 läuft. Oberteil 3 und Unterteil 4 sind an der Fußspitze 5 sowie im Bereich der Ferse 6 über jeweils eine Umlenkung 7, 8 einstückig miteinander verbunden, nachdem das Bauteil 2 wie ausgeführt einstückig und geschlossen ist, mithin also keinerlei Unterbrechungen aufweist. Die Umlenkungen 7, 8 betragen im Bereich der Fußspitze nahezu 180°, im Bereich der Ferse etwas weniger. Grundsätzlich sollte eine Umlenkung im Bereich zwischen 135° - 180° liegen.

Am Oberteil 3 ist ferner ein Befestigungsabschnitt 9 realisiert, an dem ein Verbindungsmittel 10, hier eine Pyramidenaufnahme, zur Verbindung mit einem Prothesenschaft vorgesehen ist. Dieser Befestigungsabschnitt 9 erstreckt sich von der Ferse 6 aus gesehen etwas in Richtung der Fußspitze 5, steht also leicht schräg. Zu diesem Zweck ist ersichtlich das Oberteil 3 entsprechend geformt, worauf im Nachfolgenden noch eingegangen wird.

Das Oberteil 3 weist ferner in seinem fersennahen Oberteilbereich 11 einen Federabschnitt auf, der über einen ersten Oberteilabschnitt 12 sowie einen zweiten Oberteilabschnitt 13 realisiert ist. Der erste Oberteilabschnitt 12 schließt unmittelbar an die Umlenkung 8 an und erstreckt sich etwas in Richtung Fußspitze. Er läuft in eine weitere Umlenkung 14, die ebenfalls im genannten Winkelbereich liegen sollte und geht sodann in den zweiten Oberteilabschnitt 13 über, der wiederum gebogen in den Befestigungsabschnitt 9 läuft. Im Befestigungsabschnitt 9 mündet ferner der dritte Oberteilabschnitt 15, der von der Fußspitze 5 herkommt.

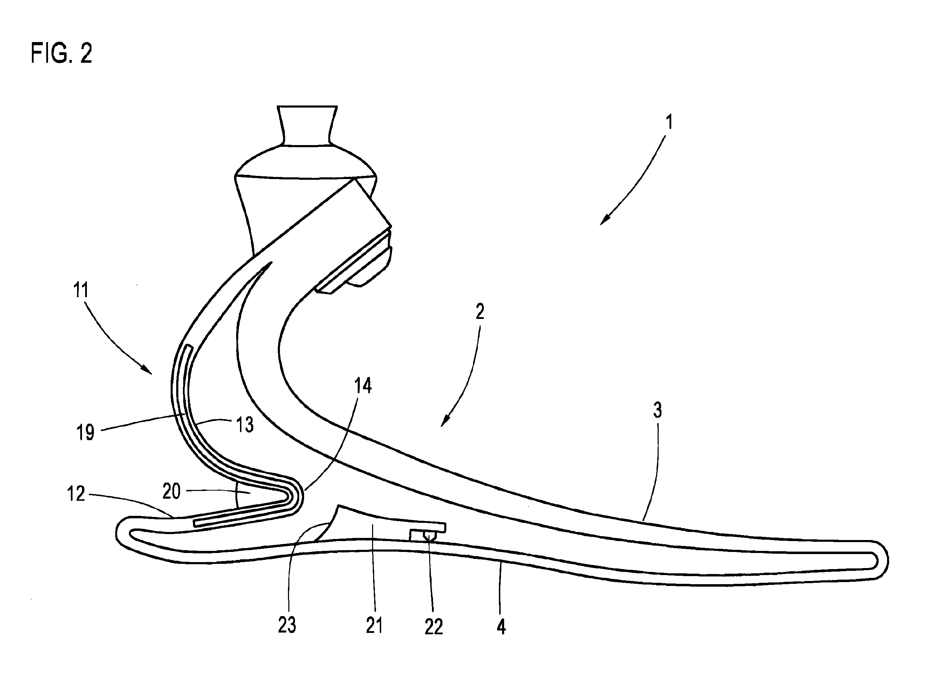

Ersichtlich ist also im fersennahen Oberteilbereich 11 ein Federabschnitt realisiert, der ein Einfedern um die Umlenkung 14 ermöglicht. Es ist quasi eine S-förmige Konfiguration (bei umgekehrter Ansicht der Prothese aus Fig. 1, wie vergleichbar in den Fig. 2 - 5 gezeigt) realisiert.

Das gesamte Bauteil 2 ist aus einem Kohlefaserverbundwerkstoff gebildet, mithin also aus einem mehrlagigen Carbonfaserbauteil, wobei über den Lagenaufbau unterschiedliche Dicken realisiert werden können, wie Fig. 1 anschaulich zeigt.

Zentraler Kern der Fußprothese 1 ist das einstückige, geschlossene Bauteil 2. Oberteil 3 und Unterteil 4 sind an den beiden äußersten Punkten, also der Fußspitze 5 und der Ferse 6 durch die bezüglich der mediolateralen Achse torsionsweiche Umlenkungen 7, 8 miteinander verbunden, worüber Oberteil 3 und Unterteil 4 auch voneinander beabstandet werden. Somit kann der Sohlenbereich, also das Unterteil 4, sich über die gesamte Fußlänge homogen verformen und tordieren. Dies gewährleistet ein homogenes Abrollen, da sich im Verlauf des Schrittzyklus, beginnend vom Aufsetzen der Ferse 6 bis hin zum Abrollen über die Fußspitze 5, die Biegesteifigkeit des sich momentan im Bodenkontakt befindlichen Bereichs des Bauteils 2 nicht sprunghaft ändert, denn das einteilige Bauteil 2, also die einteilige Feder bietet eine ununterbrochene Federwirkung. Dass heißt, dass der Energiefluss, resultierend aus der Krafteinleitung durch Aufsetzen auf dem Boden und Belasten der Fußprothese 1, nicht durch irgendwelche das Oberteil 3 mit dem Unterteil 4 starr verbindende Verbindungspunkte unterbrochen wird. Vielmehr kann die gesamte Prothesenlänge zur Federbewegung genutzt werden, d. h., dass eine maximale Federlänge respektive maximale Federwege gegeben sind. Denn Oberteil 3 und Unterteil 4 sind lediglich über die Umlenkungen 7, 8 miteinander verbunden.

Diese grundsätzliche Torsionsweichheit führt zu einer ausgewogenen Pro- und Supinationsmöglichkeit und ermöglicht so eine gute Anpassung an verschiedene Untergründe respektive auch an Bodenunebenheiten.

Bei vertikaler Krafteinleitung muss die Fußprothese gleichmäßig einfedern. Der Anbindungspunkt der Fußprothese an den Schaft und damit an das Bein des Amputierten erfolgt über das Befestigungsmittel 10. Dieses definiert letztlich auch die beiden Hebel respektive Hebellängen, die zwischen diesem Befestigungspunkt und der Fußspitze 5 und der Ferse 6 gegeben sind. Diese beiden Hebellängen sind über die beiden Pfeile P1 und P2 gezeigt. Ersichtlich ist der zur Fußspitze laufende Hebel gemäß Pfeil P1 um ein mehrfaches länger als der zur Ferse 6 laufende Hebel gemäß Pfeil P2. D. h., dass die Hebelverhältnisse zwischen der Pyramidenaufnahme und der vorderen und hinteren Umlenkungen 7, 8 unterschiedlich sind. Um dieses Hebelmissverhältnis auszugleichen dient insbesondere die Ausgestaltung des Oberteilbereichs 11 mit der Umlenkung 14 respektive den beiden Oberteilabschnitten 12, 13, die den dortigen Federabschnitt realisieren.

Wie Fig. 1 ferner zu entnehmen ist, ist am ersten Oberteilabschnitt 12 außenseitig ein Elastomerelement 16 angeordnet. Federt der über die S-Konfiguration gebildete Federabschnitt hinreichend weit ein, so kommt der zweite Oberteilabschnitt 13 in Anlage an das Elastomerelement 16, seine weitere Bewegung wird gedämpft. Hierüber kann die Härte des Fersenbereichs variiert werden.

Ferner ist ein Elastomerelement 17 zwischen dem zweiten Oberteilabschnitt 13 und dem umgebogenen Teil des dritten Oberteilabschnitts 15 im Übergang zum Befestigungsabschnitt 9 vorgesehen. Auch dieses dient der Einstellung der Federhärte in diesem Bereich.

Schließlich ist am Unterteil 4 ein drittes Elastomerelement 18 vorgesehen, das länglich ist und sich über weit die Hälfte der Unterteillänge erstreckt. Es liegt zwischen dem Unterteil 4 und dem beabstandeten Oberteil 3. Bei einer Einfederbewegung nähert sich der erste Oberteilabschnitt 12 wie auch der zweite Oberteilabschnitt 15 diesem Elastomerelement 18 und gelangen bei weiterer Einfederung in Anlage, d. h., dass die weitere Einfederbewegung wiederum gedämpft wird. Durch die jeweilige Anlage eines Bauteilabschnitts insbesondere an den Elastomerelementen 16 und 18 kommt es folglich zu einer belastungsabhängigen Widerstandserhöhung und damit einer Aufhärtung der Prothese, wobei je nach Dicke des jeweiligen Elastomerelements 16, 18 der Zeitpunkt der Aufhärtung eingestellt werden kann.

Die Figuren 2 - 5 zeigen eine weitere Ausführungsform einer erfindungsgemäßen Fußprothese 1, wobei für gleiche Bauteile gleiche Bezugszeichen verwendet werden.

Fig. 2 zeigt (in umgekehrter Ansicht wie Fig. 1) die Fußprothese 1, die von ihrer Grundform der Fußprothese 1 aus Fig. 1 entspricht.

Hier ist im Oberteilbereich 11, der fersenseitig den zusätzlichen Federabschnitt bildet, in das Kohlefaserverbundlaminat eine Elastomereinlage 19 integriert. Diese erstreckt sich vom ersten Oberteilabschnitt 12 über die Umlenkung 14 in den zweiten Oberteilabschnitt 13. Hierüber kann folglich die Biegesteifigkeit respektive Federeigenschaft in diesem Bereich eingestellt werden. Die Elastomereinlage 19 wird bei der Herstellung des Bauteils 2 bereits integriert.

Ersichtlich ist im Bereich der Umlenkung 14 ein weiteres Elastomerelement 20 integriert, das die Bewegung des ersten Oberabschnitts 12 relativ zum zweiten Oberteilabschnitt 13 bedämpft.

Wie Fig. 2 zeigt, ist am Unterteil 3, auch hier wiederum innenseitig, ein Befestigungselement 21 angeordnet, an dem, worauf nachfolgend noch gezeigt wird, unterschiedliche Elastomereinlagen fixiert werden können. Das Befestigungselement 21 weist einen Rastvorsprung 22 auf, der hier in Richtung des Unterteils 4 gerichtet ist. An ihm kann, worauf nachfolgend noch eingegangen wird, ein lösbares Elastomerelement fixiert werden.

An der anderen Seite ist eine halbrunde Gegenlagerfläche 23 realisiert, die der Abstützung eines im Bereich der Umlenkung 14 lösbar bringbaren Elastomerelements dient.

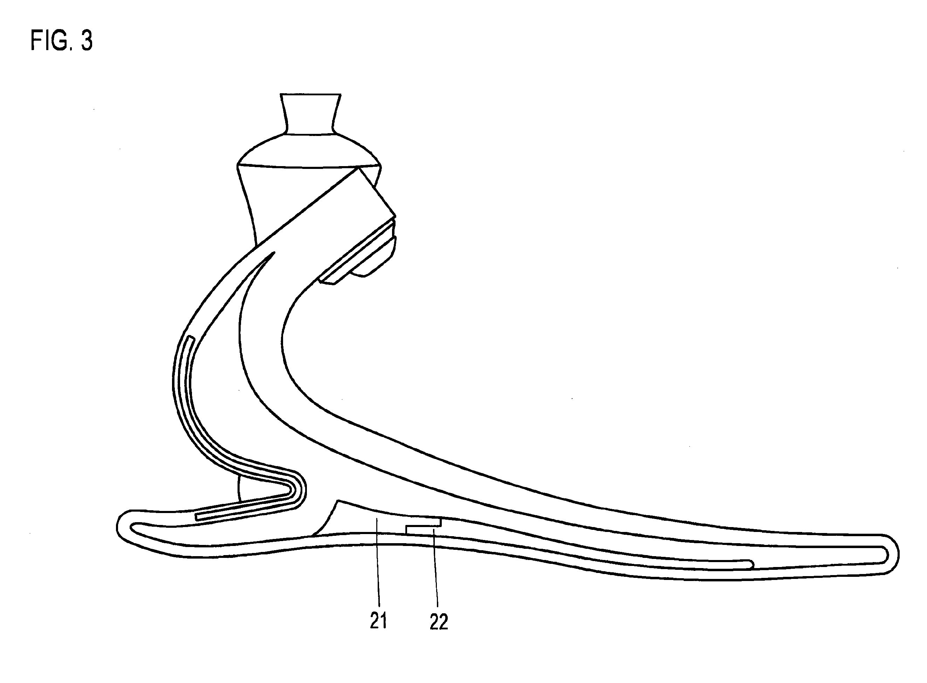

Fig. 3 zeigt die Fußprothese 1 aus Fig. 2, wobei an dem Befestigungselement 21 ein lösbares Elastomerelement 24 angeordnet ist. Dieses weist eine nicht näher gezeigte Rastaufnahme auf, in die der Rastvorsprung 22 des leicht elastischen Befestigungselements 21 eingreift. Zum Fixieren kann beispielsweise der den Rastvorsprung 22 tragende Abschnitt des Befestigungselements 21 leicht angebogen werden, sodass die Elastomereinlage 24 eingeschoben und der Rastvorsprung 22 nach Entlastung in die Rastaufnahme einrasten kann. Dieses Elastomerelement 24 kann beliebig lang sein, es kann auch, anders als in Fig. 3 gezeigt, profiliert sein, beispielsweise mit zum Oberteil ragenden dreieckförmigen Vorsprüngen oder einer grundsätzlichen Wellenform etc. Es bietet in jedem Fall dem Anwender die Möglichkeit, die Härte in diesem Bereich variieren zu können, indem das Elastomerlement 24 entweder eingesetzt oder entnommen wird, wie natürlich auch unterschiedliche harte oder lange Elastomerlemente eingesetzt werden können.

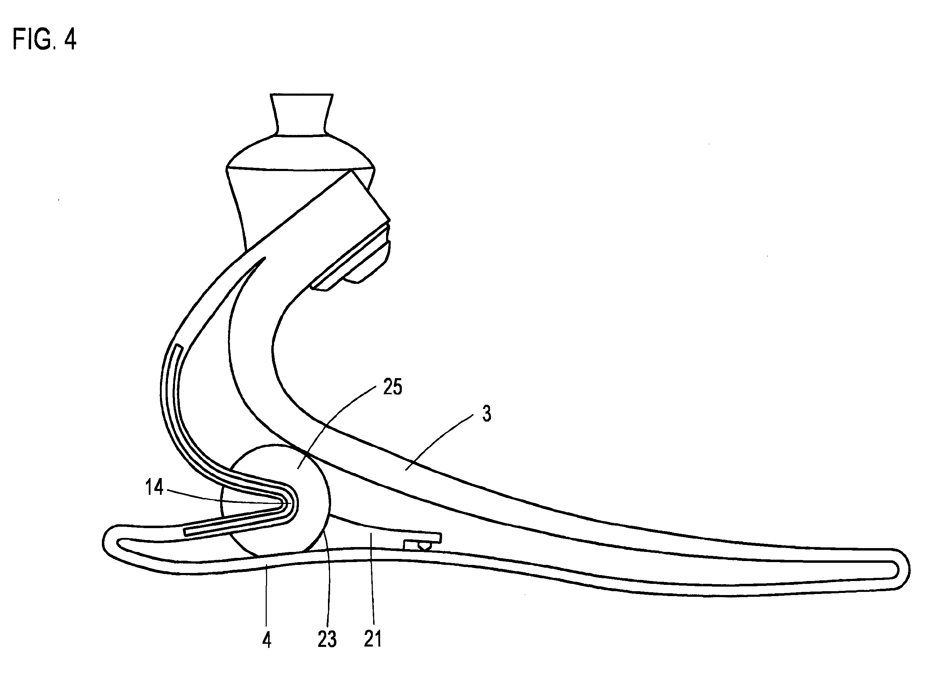

Fig. 4 zeigt die Fußprothese 1 aus Fig. 1, bei der ein weiteres Elastomerelement 25 eingesetzt ist, das im gezeigten Beispiel rundlich ist und die Umlenkung 14 umgreift. Es ist am Gegenlagerabschnitt 23 des Befestigungselements 21 gegengelagert. Es liegt unterseitig am Unterteil 4, oberseitig am Oberteil 3 an, ist mit diesen jedoch nur über die Anlage, jedoch nicht fest verbunden. Ersichtlich dämpft es die Einfederbewegung bei Belastung der Fußprothese 1, wenn sich das Oberteil 3 in Richtung des Unterteils 4 bewegt. Auch hier kann durch unterschiedlich harte Elastomerelemente 25 die Prothesenhärte variiert werden. Wird das Elastomerelement 25 etwas schmäler gewählt, so dass es das Oberteil 3 nicht kontaktiert, so kommt es erst bei hinreichendem Einfedern zur Anlage und damit zur Aufhärtung.

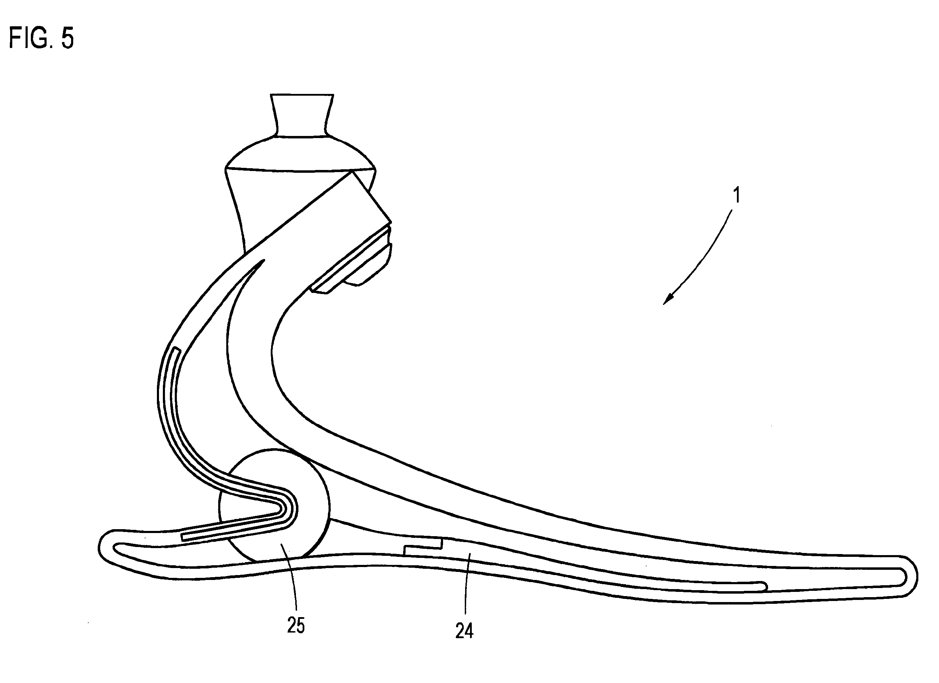

Fig. 5 zeigt schließlich die Fußprothese 1, wobei hier sowohl das Elastomerelement 24 als auch das Elastomerelement 25 eingesetzt sind.

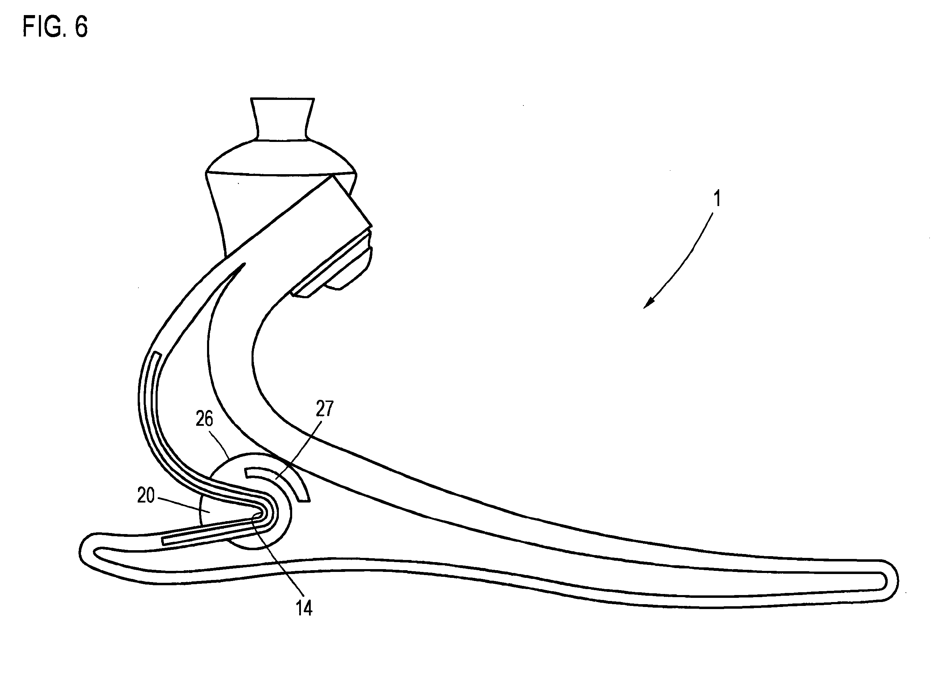

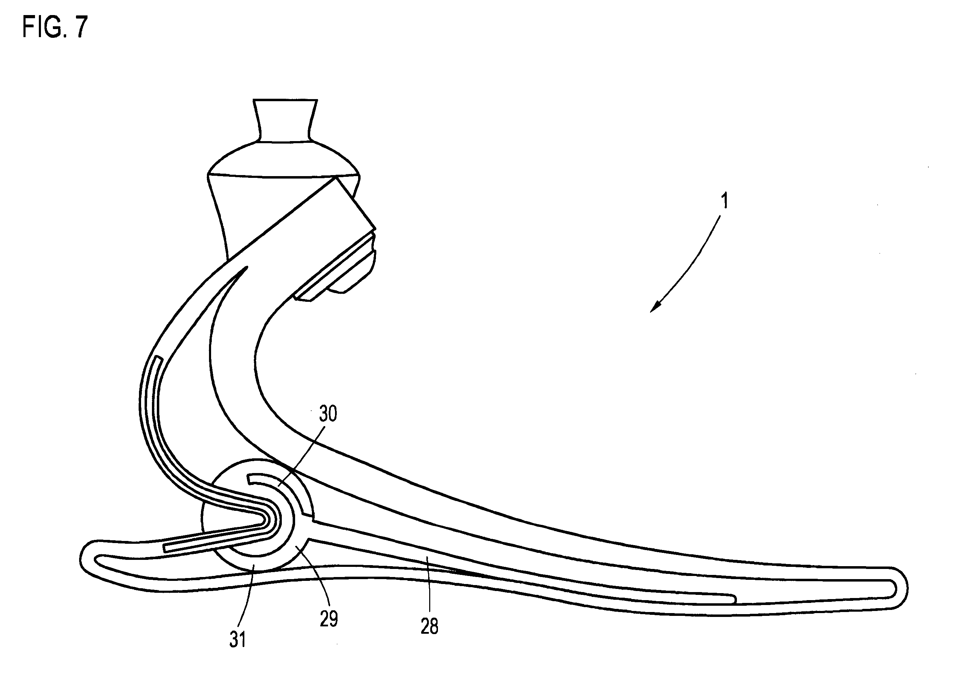

Die Figuren 6 und 7 zeigen schließlich noch eine weitere Ausgestaltung einer erfindungsgemäßen Fußprothese, die sich wiederum hinsichtlich der verwendeten Elastomerelemente von den zuvor beschriebenen Ausführungsformen unterscheidet. Fig. 6 zeigt eine Fußprothese 1, wie sie in ihrer Grundform den zuvor beschriebenen Ausführungsformen entspricht. Rückwärtig im Fersenbereich ist wiederum ein Elastomerelement 20 verklebt, gegenüberliegend, wiederum die Umlenkung 14 umgreifend, ist ein Elastomerelement 26 angeordnet, das dem Elastomerelement 25 ähnlich ist, jedoch eine etwas andere Geometrie aufweist. Es weist einen gebogenen Schlitz 27 auf, der der Fixierung eines von der Seite her einsteckbaren, am Elastomerelement 26 halterbaren länglichen Elastomerelement 28 dient, wie in Fig. 7 gezeigt. Das Elastomerelement 28 weist einen rundlichen Befestigungsabschnitt 29 auf, der mit einem Abschnitt 30 in den rundlichen Spalt 27 eingreift und mit seinem anderen Abschnitt 31 das Elastomerelement 26 unterseitig umgreift und auf dem Unterteil aufgelagert ist. Es ist hier also eine besondere Haltegeometrie realisiert, die ein seitliches Einstecken des Elastomerelements 28 ermöglicht.

## Patentansprüche

1. Fußprothese, umfassend ein Oberteil und ein beim Laufen bodenseitig aufsetzendes Unterteil, die übereinander und voneinander beabstandet verlaufen, an der Fußspitze und der Ferse miteinander verbunden sind, und, eine Feder bildend, beim Laufen relativ zueinander bewegen, wobei das Oberteil (3) und das Unterteil (4) mittels eines einstückigen, geschlossenen Bauteils (2) aus einem Kohlefaserverbundwerkstoff gebildet sind, **dadurch gekennzeichnet, dass** das Oberteil (3) und das Unterteil (4) über jeweils eine im Bereich zwischen 135° - 180° verlaufende Umlenkung (7, 8) an der Fußspitze (5) und der Ferse (6) ineinander übergehen.

2. Fußprothese nach Anspruch 1, **dadurch gekennzeichnet, dass** das Bauteil (2) im fersennahen Bereich des Oberteils eine weitere Umlenkung (14) aufweist, derart, dass ein oberhalb des Unterteils (4) verlaufender erster Oberteilabschnitt (12) über die Umlenkung (14) in einen oberhalb des ersten Abschnitts (12) verlaufenden zweiten Oberteilabschnitt (13) übergeht.

3. Fußprothese nach Anspruch 2, **dadurch gekennzeichnet, dass** im Bereich der Umlenkung (14) an einem oder beiden Oberteilabschnitten (12, 13) ein Elastomerelement (16) eingebracht ist.

4. Fußprothese nach Anspruch 2 oder 3, **dadurch gekennzeichnet, dass** im Bereich zwischen dem Oberteil (3) und dem Unterteil (4) ein die Umlenkung (14) umgreifendes, zumindest beim Laufen das Oberteil (3) mit dem Unterteil (4) koppelndes Elastomerelement (25) vorgesehen, insbesondere lösbar einsetzbar ist.

5. Fußprothese nach einem der vorangehenden Ansprüche, **dadurch gekennzeichnet, dass** das Oberteil (3) im fersennahen Bereich, insbesondere im Bereich des ersten und/oder zweiten Oberteilabschnitts (12, 13), eine Elastomereinlage (19) im Kohlefaserverbundwerkstoff aufweist.

6. Fußprothese nach einem der vorangehenden Ansprüche, **dadurch gekennzeichnet, dass** das Bauteil (2) im fersennahen Bereich, insbesondere mit dem zweiten Oberteilabschnitt (13), gebogen in Richtung der Fußspitze (5) verläuft und mit dem von der Fußspitze (5) kommenden Oberteilabschnitt (15), der im fersennahen Bereich ebenfalls in Richtung der Fußspitze (5) umgebogen verläuft, unter Bildung eines Befestigungsabschnitts (9) für ein Verbindungsmittel (10), insbesondere eine Pyramidenaufnahme, zusammenläuft.

7. Fußprothese nach Anspruch 6, **dadurch gekennzeichnet, dass** im Bereich der Verbindung beider Oberteilabschnitte (13, 15) ein beide verbindendes Elastomerelement (17) eingebracht ist.

8. Fußprothese nach einem der vorangehenden Ansprüche, **dadurch gekennzeichnet, dass** im Bereich zwischen dem Oberteil (3) und dem Unterteil (4) ein Befestigungselement (21) für ein vorzugsweise lösbar einsetzbares und beim Laufen das Oberteil (3) mit dem Unterteil (4) koppelndes Elastomerelement (24) vorgesehen ist.

9. Fußprothese nach Anspruch 8, **dadurch gekennzeichnet, dass** das Befestigungselement (21) einen Rastvorsprung (22), der vorzugsweise zum Unterteil (4) gerichtet ist, aufweist, der in eine an dem einsetzbaren Elastomerelement (24) vorgesehene Rastaufnahme eingreift.

## Claims

1. Foot prosthesis, comprising an upper part, and a lower part that is placed on the ground when walking, which parts extend one above the other and at a distance from each other, are connected to each other at the forefoot and the heel and, forming a spring, move relative to each other during walking, the upper part (3) and the lower part (4) being formed by means of a one-piece, closed component (2) made from a carbon fiber composite, **characterized in that** the upper part (3) and the lower part (4) merge into each other at the forefoot (5) and the heel (6), in each case via a deflection (7, 8) in the range of between 135° and 180°.

2. Foot prosthesis according to Claim 1, **characterized in that** the component (2), in the area of the upper part near the heel, has a further deflection (14), in such a way that a first upper-part portion (12) extending above the lower part (4) merges via the deflection (14) into a second upper-part portion (13) extending above the first portion (12).

3. Foot prosthesis according to Claim 2, **characterized in that**, in the area of the deflection (14), an elastomer element (16) is fitted on one or both upper-part portions (12, 13).

4. Foot prosthesis according to Claim 2 or 3, **characterized in that**, in the area between the upper part (3) and the lower part (4), an elastomer element (25) that engages around the deflection (14) and couples the upper part (3) to the lower part (4), at least during walking, is provided, in particular can be inserted releasably.

5. Foot prosthesis according to one of the preceding claims, **characterized in that** the upper part (3), in the area near the heel, in particular in the area of the first and/or second upper-part portion (12, 13), has an elastomer inlay (19) within the carbon fiber composite.

6. Foot prosthesis according to one of the preceding claims, **characterized in that** the component (2), in the area near the heel, extends in particular with the second upper-part portion (13) curved in the direction of the forefoot (5) and converges with the upper-part portion (15), which comes from the forefoot (5) and in the area near the heel is likewise curved in the direction of the forefoot (5), in order to form a securing portion (9) for a connection means (10), in particular a pyramid adapter.

7. Foot prosthesis according to Claim 6, **characterized in that**, in the area of the connection of the two upper-part portions (13, 15), an elastomer element (17) is fitted that connects both portions.

8. Foot prosthesis according to one of the preceding claims, **characterized in that**, in the area between the upper part (3) and the lower part (4), a securing element (21) is provided for a preferably releasably insertable elastomer element (24) which, during walking, couples the upper part (3) to the lower part (4).

9. Foot prosthesis according to Claim 8, **characterized in that** the securing element (21) has a latching projection (22) which is preferably directed toward the lower part (4) and which engages in a latching recess provided on the insertable elastomer element (24).

## Revendications

1. Prothèse du pied, comprenant une partie supérieure et une partie inférieure reposant vers le sol lors de la marche, qui sont placées l'une au-dessus de l'autre et à distance l'une de l'autre, qui sont reliées l'une à l'autre à la pointe du pied et au talon et qui, en faisant ressort, se déplacent l'une par rapport à l'autre lors de la marche, dans laquelle la partie supérieure (3) et la partie inférieure (4) sont formées au moyen d'un composant fermé d'une seule pièce (2) en un matériau composite de fibres de carbone, **caractérisée en ce que** la partie supérieure (3) et la partie inférieure (4) se prolongent l'une dans l'autre à la pointe du pied (5) et au talon (6) respectivement par l'intermédiaire d'une déviation (7, 8) s'étendant dans une plage comprise entre 135° et 180°.

2. Prothèse du pied selon la revendication 1, **caractérisée en ce que** le composant (2) présente, dans la région proche du talon de la partie supérieure, une autre déviation (14), de telle manière qu'une première section (12) de la partie supérieure s'étendant au-dessus de la partie inférieure (4) se prolonge, par l'intermédiaire de la déviation (14), dans une deuxième section (13) de la partie supérieure s'étendant au-dessus de la première section (12).

3. Prothèse du pied selon la revendication 2, **caractérisée en ce qu'**un élément élastomère (16) est introduit dans la région de la déviation (14) sur une ou sur les deux section(s) (12, 13) de la partie supérieure.

4. Prothèse du pied selon la revendication 2 ou 3, **caractérisée en ce qu'**il est prévu, dans la région comprise entre la partie supérieure (3) et la partie inférieure (4), un élément élastomère (25) entourant la déviation (14) et couplant la partie supérieure (3) à la partie inférieure (4) au moins lors de la marche, et qui peut en particulier être inséré de façon séparable.

5. Prothèse du pied selon l'une quelconque des revendications précédentes, **caractérisée en ce que** la partie supérieure (3) présente dans la région proche du talon, en particulier dans la région de la première et/ou de la deuxième section (12, 13) de la partie supérieure, un insert élastomère (19) dans le matériau composite de fibres de carbone.

6. Prothèse du pied selon l'une quelconque des revendications précédentes, **caractérisée en ce que** le composant (2) s'étend, dans la région proche du talon, en particulier avec la deuxième section (13) de la partie supérieure, sous forme courbée en direction de la pointe du pied (5) et converge avec la section (15) de la partie supérieure venant de la pointe du pied (5), qui s'étend sous forme recourbée dans la région proche du talon également en direction de la pointe du pied (5), en formant une section de fixation (9) pour un moyen de jonction (10), en particulier un logement pyramidal.

7. Prothèse du pied selon la revendication 6, **caractérisée en ce qu'**un élément élastomère (17) est introduit dans la région de la liaison des deux sections (13, 15) de la partie supérieure de façon à relier les deux.

8. Prothèse du pied selon l'une quelconque des revendications précédentes, **caractérisée en ce qu'**il est prévu dans la région située entre la partie supérieure (3) et la partie inférieure (4) un élément de fixation (21) pour un élément élastomère (24) insérable de préférence de façon séparable et couplant la partie supérieure (3) à la partie inférieure (4) lors de la marche.

9. Prothèse du pied selon la revendication 8, **caractérisée en ce que** l'élément de fixation (21) présente une saillie d'encliquetage (22), qui est de préférence dirigée vers la partie inférieure (4), qui s'engage dans un logement d'encliquetage prévu sur l'élément élastomère insérable (24).
